# EUROPÄISCHE PATENTSCHRIFT

(11) **EP 0 711 170 B1**
(45) Veröffentlichungstag und Bekanntmachung des Hinweises auf die Patenterteilung: **08.04.1998**
(21) Anmeldenummer: 94923735.8
(22) Anmeldetag: 13.07.1994
(51) Int. Cl.: A61K 35/14

(54) **T-LYMPHOZYTEN ENTHALTENDE PHARMAZEUTISCHE ZUSAMMENSETZUNG ZUR BEHANDLUNG VON INFEKTIONEN**
T-LYMPHOCYTE-CONTAINING PHARMACEUTICAL COMPOSITION FOR TREATING INFECTIONS
COMPOSITION PHARMACEUTIQUE CONTENANT DES LYMPHOCYTES T UTILISEE POUR LE TRAITEMENT D'INFECTIONS

(30) Priorität: 29.07.1993 DE 4325959; 16.06.1994 DE 4422020
(43) Veröffentlichungstag der Anmeldung: 15.05.1996
(73) Patentinhaber: Heising, Bernhard, 14059 Berlin (DE); Heising, Edelgard, 14059 Berlin (DE); Heising, Cathrin, 14059 Berlin (DE); Heising, Franziska, 14059 Berlin (DE)
(72) Erfinder: Heising, Bernhard, 14059 Berlin (DE); Heising, Edelgard, 14059 Berlin (DE); Heising, Cathrin, 14059 Berlin (DE); Heising, Franziska, 14059 Berlin (DE)
(74) Vertreter: Ziebig, Marlene, Dr. Dipl.-Chem.
(86) Internationale Anmeldenummer: EP9402313
(87) Internationale Veröffentlichungsnummer: WO9503812

(56) Entgegenhaltungen:
- EP-A- 0 060 472
- EP-A- 0 202 642
- EP-A- 0 260 714
- DIABETES, Bd.38, Januar 1989, ALEXANDRIA VA, VSA Seiten 24 - 30 D. BURSTEIN ET AL. 'Prevention of diabetes in BB/Wor rat by single transfusion of spleen cells.'
- IMMUNOLOGY, Bd.52, Nr.3, Juli 1984, OXFORD, GB Seiten 404 - 410 M. JEGGO ET AL. 'A study of the role of cell-mediated immunity in bluetongue virus infection in sheep, using cellular adoptive transfer techniques.'
- L. MORETTA ET AL., IN: 'ENCYCLOPEDIA OF IMMUNOLOGY (Ed. I. Roitt)' 1992 , ACADEMIC PRESS , LONDON, GROSSBRITANNIEN, SEITEN 321-325 siehe Tabelle 1 siehe Seite 323, linke Spalte, Zeile 19 - Zeile 22

## Beschreibung

Die Erfindung betrifft eine pharmazeutische Zusammensetzung, die T-Lymphozyten als Wirkstoff enthält, ein Verfahren zur Herstellung dieser pharmazeutischen Zusammensetzung und deren Verwendung zur Herstellung von Arzneimitteln zur Behandlung von viralen und bakteriellen Infektionen.

Infektionskrankheiten, die durch Bakterien oder Viren verursacht werden, werden heute zu einem großen Teil mit Chemotherapeutika behandelt. Antibakterielle Chemotherapeutika sind Stoffwechselprodukte von Mikroorganismen oder deren halbsynthetische Derivate (Antibiotika) oder synthetisch hergestellte Verbindungen (Sulfonamide).

Als Chemotherapeutika, die durch Hemmung der Virusproduktion eine Therapie virusbedingter Infektionen ermöglichen, werden heute bevorzugt Nukleosid-Analoga eingesetzt, die die virusspezifischen Enzyme (Polymerasen) hemmen.

Ein wesentlicher und allgemein bekannter Nachteil der Chemotherapeutika ist, daß bei längerer und wiederholter Behandlung Resistenzen auftreten können. Außerdem wird häufig über Nebenwirkungen berichtet.

Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen.

So steht derzeit beispielsweise für die Behandlung von AIDS fast ausschließlich das 3'-Azido-3'-deoxy-thymidin (AZT) zur Verfügung. AZT ist jedoch durch bereits im therapeutischen Bereich auftretende, sehr schwere Toxizitäten charakterisiert (Hirsch, M.S. (1988) J.Infec. Dis. 157, 427-431).

Aufgabe der vorliegenden Erfindung war es deshalb, ein neues Therapeutikum zu entwickeln, das zur Behandlung viraler, insbesondere retroviraler, und bakterieller Infektionen eingesetzt werden kann, ohne daß bei längerer oder wiederholter Behandlung Resistenzen oder NeNebenwirkungen auftreten und pharmakologisch relevante Dosen cytotoxisch sind.

Überraschend wurde nun gefunden, daß eine äußerst einfach herstellbare und kostengünstige pharmazeutische Zusammensetzung, die T-Lymphozyten aus Säugetierblut, - milz oder -thymusdrüse oder auch aus Eigenblut als Wirkstoff enthält, zur Behandlung viraler und bakterieller Infektionen sehr gut geeignet und schnell wirksam ist.

Die in der Zusammensetzung enthaltenen T-Lymphozyten werden aus Säugetiervollblut, z. B. von Kaninchen, Lämmern oder Schweinen, gewonnen, indem das Saugetiervollblut, dem gegebenenfalls wäßriges Trinatriumcitrat als Antikoagulans (0,11 mol/l; 9 Teile Blut, 1 Teil Citrat) zugesetzt wird, einer Zentrifugation von ca. 7 bis 8 Minuten bei ca. 4000 Umdrehungen/Minute unterworfen wird, und die Leukozytengrenzschicht separiert wird. Durch Auftrennen der Leukozyten über Nylonwolle wird eine angereicherte T-Zellsuspension erhalten. Analog erfolgt die Gewinnung aus Eigenblut. Dem Eigenblut werden vorzugsweise ebenfalls wäßriges Trinatriumcitrat und Dextrose zugefügt.

Eine weitere Möglichkeit der Leukozytengewinnung aus Säugetiervollblut oder Eigenblut besteht darin, daß man das Vollblut, dem gegebenenfalls wäßriges Trinatriumcitrat (0,11 mol/l, 9 Teile Blut, 1 Teil Citrat) zugesetzt wurde, durch einen kommerziell erhältlichen Zellulosefilter schickt, diesen mit PBS auswäscht, die erhaltene Leukozyten-/ Thrombozytensuspension anschließend 7 bis 8 Minuten bei 700 Umdrehungen/Minute zentrifugiert, um Thrombozyten und Leukozyten zu separieren. Die Auftrennung der Leukozyten erfolgt wiederum über Nylonwolle. Mit diesem Verfahren wird eine besonders hoch konzentrierte T-Lymphozyten-suspension erhalten.

Selbstverständlich lassen sich die Leukozyten aus Säugetier- oder Eigenblut auch mit dem bekannten Verfahren der Leukopherese separieren.

Alternativ kann die erfindungsgemäße T-Lymphozytensuspension auch aus Säugetiermilz oder aus der Säugetierthymusdrüse gewonnen werden, indem man die Milz bzw. die Drüse mit PBS (Phosphatgepufferte, physiologische Kochsalz-Lösung), dem gegebenenfalls Trinatriumcitrat und Dextrose zugesetzt wurde, auswäscht, die erhaltene Lösung ca. 7 bis 8 Minuten zentrifugiert und die Leukozytenschicht separiert. Durch Auftrennen der Leukozyten über Nylonwolle wird wiederum eine angereicherte T-Zellsuspension erhal-ten.

Zur oralen Applikation werden 20 bis 50 g der T-Zellsuspension in vorzugsweise 100 bis 300 ml einer nicht denaturierenden Flüssigkeit gegeben. Als Flüssigkeit kann im einfachsten Fall Wasser dienen, aber auch Säfte oder Instantsuppe kommen zur Anwendung. Die Verwendung von Instantsuppe hat den großen Vorteil, daß die Zugabe von Geschmackskorrigentien oder Süßstoffen zur besseren Akzeptanz des Arzneimittels durch den Patienten entfallen kann.

Die Herstellung einer Darreichungsform der erfindungsgemäßen pharmazeutischen Zusammensetzung unter Zuhilfenahme von z. B. Instantsuppe erfolgt durch Zubereitung der Instantsuppe, Abkühlen dieser auf Raumtemperatur und Einrühren der entsprechenden Menge T-Zellsuspension. Vorzugsweise werden dabei 20 bis 50 g T-Zellsuspension in 250 ml Instantsuppe gelöst. Die T-Zellsuspension wird vorzugsweise im gefrorenen Zustand (portioniert) aufbewahrt und unaufgetaut in die Flüssigkeit eingerührt. Dadurch kommt es zum Platzen der T-Zellen. Wird eine frisch zubereitete T-Zellsuspension zur Herstellung der erfindungsgemäßen Darreichungsform verwendet, so muß die Suspension unter kräftigem Rühren (Mixer) in die Flüssigkeit eingebracht werden, um die T-Zellen zu zerstören. Voraussetzung für die erfindungsgemäß festgestellte hohe und schnelle Wirksamkeit ist, daß die T-Zellen bereits im geplatzten Zustand aufgenommen werden.

Die T-Lymphozytensuspension kann auch rektal als Suppositorium oder als Klistier appliziert werden. Bei der Verabreichung als Klistier wird die T-Zellsuspension vorzugsweise unverdünnt bzw. zur besseren Handhabbarkeit mit wenig Wasser verdünnt gegeben.

Die verabreichte Dosis hängt vom Alter, der Gesundheit und dem Gewicht des Empfängers ab.

Üblicherweise beträgt die tägliche Dosis 20 bis 50 g der T-Zellsuspension, die in einer Applikation gegeben werden sollte.

Eine erfindungsgemäße Variante der pharmazeutischen Zusammensetzung betrifft auch eine T-Zellsuspension aus Eigenblut, die in analoger Weise wie die T-Zellsuspension aus Säugetierblut hergestellt wird, jedoch mittels Zerstäuber inhaliert werden muß. Hierbei muß die Applikation jedoch über den Tag verteilt ca. dreimal erfolgen, wobei vorzugsweise nicht mehr als 10 ml/Tag verabreicht werden sollen.

Anaphylaktische Schocks, wie sie bei der Verabreichung von Vollblut oder Vollblutpräparaten beobachtet wurden, treten bei der oralen Applikation der erfindungsgemäßen Zusammensetzung nicht auf.

Die erfindungsgemäße pharmazeutische Zubereitung besitzt wertvolle pharmakologische Eigenschaften. Es zeigte sich, daß bei bakteriellen oder Virusinfektionen bereits nach fünftägiger Applikation keinerlei Krankheitssymptome mehr vorhanden waren. In schweren Fällen, vor allem bei persistierenden Infektionen, kann die T-Zellsuspension bis zu 30 Tagen gegeben werden, ohne daß Nebenwirkungen auftreten. Die wiederholte Anwendung der erfindungsgemäßen pharmazeutischen Zubereitung führt zu keinerlei Resistenzen oder toxischen Nebenwirkungen.

Mit der aus Säugetiervollblut hergestellten erfindungsgemäßen pharmazeutischen Zubereitung wurden bisher Probanden behandelt, die an viralen und bakteriellen Infektionen litten, darunter solche mit Gürtelrose, Gesichtsherpes, mit Pfeifferschem Drüsenfieber (Epstein-Barr-Virus) und Aids.

Auch ein an einer bakteriellen Infektion erkrankter Hund wurde mit dem erfindungsgemäßen Mittel therapiert.

Die Wirkung der erfindungsgemäßen Zubereitung bei Retrovirusinfektionen wurde an Aids-Probanden mit Hilfe des HIV-p24-Antigen-Elisa festgestellt.

Offensichtlich interferiert die erfindungsgemäße Zusammensetzung mit retroviral bedingten Erkrankungen oder deren Symptomen, ohne die normal ablaufenden natürlichen Körperfunktionen zu beeinflussen.

Gegenstand der Erfindung ist deshalb auch die Verwendung der erfindungsgemäßen T-Lymphozytensuspension zur Herstellung von Arzneimitteln zur Behandlung viraler und bakterieller Infektionen, insbesondere von retroviralen Infektionen wie AIDS und ARC (AIDS-related-complex)

Die Erfindung soll nachfolgend durch Ausführungsbeispiele näher erläutert werden, ohne sie darauf einzuschränken.

### Ausführungsbeispiele

### 1. Beispiel

Die erfindungsgemäße pharmazeutische Zusammensetzung, bestehend aus 30 g T-Zellsuspension aus Kaninchenblut in 250 ml Instant-Suppe, wurde wiederholt an eine 48-jährige Frau mit einer Virusinfektion und (4 Monate später) einem grippalen Infekt verabreicht:

### a) Virusinfektion

- Beginn der Infektion mit Anzeichen von Leistungsabfall und Schwäche, starke Kopfschmerzen, Fieber, Fieberzäpfchen genommen, Besserung ohne Fieber nach 2 Tagen,
- ca. 8 Tage später, immer noch starke Kopfschmerzen, wieder leichtes Fieber, Schwitzen, Frösteln, Mattigkeit, nach 4 Tagen wieder Besserung,
- 13 Tage nach den ersten Anzeichen der Infektion Arztbesuch, da jetzt starke Schwächung, leichtes Fieber, festsitzender, harter Husten, festsitzender Schnupfen, daher Schlafstörungen und ständige Müdigkeit,
Arztdiagnose nach Blutentnahme: Virusinfektion eines äußerst seltenen Virus, mit schlechten Leberwerten,
- Behandlung:: 3 x tgl. inhallieren, Nasentropfen, Hustentropfen, 3 x tgl. 6 Tabl. Wobenzym N (Enzyme), 3 x tgl. 2 Tabl. Gripp-Heel, (Grippe), 2 x tgl. 3 Tropfen Grippe Comp.,
- - am 9. und 10. Tag (nach Arztbesuch): trotz der genannten Behandlung totale Schwäche, unerträglicher Hustenreiz
- - am 11. Tag (nach Arztbesuch): Verabreichung der ersten erfindungsgemäßen Zusammensetzung
- - am 12. Tag: Verabreichung der 2. Zusammensetzung, nachts zum ersten Mal durchgeschlafen, nur noch wenig Husten, Schnupfen fast verschwunden
- - am 13. Tag: Verabreichung der 3. Zusammensetzung, Husten und Schnupfen vollkommen verschwunden allgemeines Befinden zu 80% wieder hergestellt
- - am 14. Tag: Verabreichung der 4. Zusammensetzung, keine Beschwerden mehr, Kopfschmerzen verschwunden, ungestörter Nachtschlaf
- - am 15. Tag: die 5. und letzte Zusammensetzung verabreicht, keinerlei Krankheitssymptome mehr.

### b) Grippaler Infekt

4 Monate später trat erneut ein starker grippaler Infekt auf, der wiederum durch 10-tägige Einnahmeder erfindungsgemäßen Zusammensetzung therapiert wurde. Bereits nach 3-tägiger Einnahme (einmal täglich abends) stellte sich Linderung ein und nach 10 Tagen war die Patientin gesund.

Der Gesundheitszustand der Patientin hat sich nach dieser zweiten Einnahme derart stabilisiert, daß sie bisher 1,5 Jahre keinen viralen oder bakteriellen Infekt mehr hatte.

### 2. Beispiel

Ein 43-jähriger, an einem grippalen Infekt leidender Patient erhielt die erfindungsgemäße Zusammensetzung gemäß Beispiel 1.

Herr R. H. klagte über allgemeine Abgeschlagenheit, Gliederschmerzen, Appetitlosigkeit und leichten Schwindel bei Lagewechsel. Am nächsten Tag kam es zum Auftreten von Schüttelfrost und febrilen Temperaturen bis axillar 38,8 °C.

### Befund:

- guter Ernährungszustand und mäßig reduzierter Allgemeinzustand; krankhaftes Aussehen; Haut blaß, warm, leicht schweißig; Schleimhäute ausreichend durchblutet; Kopf frei beweglich, geringer Kopfschmerz über beiden Kieferhöhlen; Zunge feucht, leicht belegt; Rachenhinterwand gering gerötet.
- Herz: o. B.
- Lunge: beidseitig belüftet, diskret verschärftes Atemgeräusch beidseitig paravertebral.
- Abdomen: o. B.
- Extremitäten: frei beweglich
- Neurologie: o. B.

Nach Verabreichung der ersten erfindungsgemäßen Zusammensetzung kam es in der folgenden Nacht zu einem starken Schwitzen. Am Morgen darauf fühlte sich der Patient schon bedeutend wohler. Nach Verabreichung der zweiten erfindungsgemäßen Zusammensetzung kam es zu einer weiteren Besserung und nach Verabreichung der dritten Zubereitung am nächsten Tag waren die oben genannten Symptome bis auf ein leichtes Husten verschwunden.

### 3. Beispiel

### Verabreichung der erfindungsgemäßen Zusammensetzung als Klistier

Ein an einer bakteriell bedingten Schneegastritis schwer erkrankter Hund wurde durch 14-tägige Gabe von täglich 20 g erfindungsgemäßer T-Zellsuspension geheilt. Die Applikation erfolgte enteral mittels Spritze.

### 4. Beispiel

### Verabreichung der erfindungsgemäßen Zusammensetzung bei Gürtelrose

Ein an Gürtelrose erkrankter Patient wurde durch die 10-tägige orale Verabreichung der erfindungsgemäßen Zusammensetzung gemäß Beispiel 1 erfolgreich therapiert.

### 5. Beispiel

### Verabreichung der erfindungsgemäßen Zusammensetzung bei Gesichtsherpes

Eine Patientin mit breitflächiger Gesichtsherpes, hervorgerufen durch das Herpes-Simplex-Virus, wurde durch 5-tägige Verabreichung der erfindungsgemäßen Zusammensetzung gemäß Beispiel 1 geheilt. Bei täglich einmaliger Einnahme klang bereits am 3. Tag die Entzündung ab und am 5. Tag war die entzündliche Fläche vollkommen abgeheilt.

### 6. Beispiel

### Verabreichung der erfindungsgemäßen Zusammensetzung an eine Patientin mit Pfeifferschem Drüsenfieber (Epstein-Barr-Virus)

Die erfindungsgemäße Zusammensetzung gemäß Beispiel 1 wurde einer Patientin mit Pfeifferschem Drüsenfieber täglich einmal verabreicht. Bereits nach der zweiten Einnahme sank das Fieber erheblich und die Patientin fühlte sich besser. Am dritten Tag war die Patientin fieberfrei und nach weiteren drei Tagen konnte sie wieder das Haus verlassen.

### 7. Beispiel

### Verabreichung der erfindungsgemäßen Zusammensetzung an einen AIDS-Patienten

Die erfindungsgemäße Zusammensetzung gemäß Beispiel 1 wurde einem AIDS-Patienten (HIV-1) 30 Tage lang täglich einmal verabreicht.

Während in dem vor Beginn der Therapie durchgeführten bekannten HIV-p24-Antigen-Test (Meßbereich 5-500 pg/ml) eindeutig p24-Antigen nachgewiesen wurde, konnte nach Durchführung der Therapie kein p24-Antigen mehr gefunden werden. Daraus wird ersichtlich, daß offensichtlich eine Hemmung der Virusvermehrung eingetreten ist.

## Patentansprüche

1. Pharmazeutische Zusammensetzung, enthaltend als Wirkstoff T-Lymphozyten,
hergestellt durch Gewinnung der Leukozyten aus Säugetiervollblut, Säugetiermilz oder Säugetierthymusdrüse oder Eigenblut, Auftrennung der Leukozyten über Nylonwolle und Gewinnung der angereicherten T-Lymphozytensuspension.

2. Pharmazeutische Zusammensetzung gemäß Anspruch 1 hergestellt durch Gewinnung der Leukozyten mittels Zentrifugation von Säugetiervollblut oder Eigenblut und Separierung der Leukozytengrenzschicht.

3. Pharmazeutische Zusammensetzung gemäß Anspruch 1, hergestellt durch Gewinnung der Leukozyten mittels Filtration von Säugetiervollblut oder Eigenblut, dem gegebenenfalls wäßrige Trinatriumcitratlösung als Antikoagulans zugesetzt ist, über einen Zellulosefilter, Auswaschen des Filters mit PBS, Zentrifugation der erhaltenen Leukozyten-/ Thrombozytensuspension und Separierung der Leukozytenschicht.

4. Pharmazeutische Zusammensetzung gemäß Anspruch 1, hergestellt durch die Gewinnung der Leukozyten aus Säugetiervollblut oder Eigenblut mittels Leukopherese.

5. Pharmazeutische Zusammensetzung gemäß Anspruch 1, hergestellt durch Gewinnung der Leukozyten durch Auswaschen von Säugetiermilz oder Säugetierthymusdrüse mit PBS, dem gegebenenfalls Trinatriumcitrat zugesetzt ist, Zentrifugation der erhaltenen Lösung und Separierung der Leukozytenschicht.

6. Verfahren zur Herstellung einer pharmazeutischen Zusammensetzung gemäß Anspruch 1, dadurch gekennzeichnet, daß man die T-Lymphozyten in eine geeignete Darreichungsform einarbeitet.

7. Verwendung einer T-Lymphozytensuspension zur Herstellung von Arzneimitteln zur Behandlung von viralen und bakteriellen Infektionen, die hergestellt wird durch Gewinnung der Leukozyten aus Säugetiervollblut, Säugetiermilz oder Säugetierthymusdrüse oder Eigenblut, Auftrennung der Leukozyten über Nylonwolle und Gewinnung der angereicherten T-Lymphozytensuspension.

8. Verwendung einer T-Lymphozytensuspension gemäß Anspruch 7 zur Herstellung von Arzneimitteln zur Behandlung von AIDS und ARC.

## Claims

1. A pharmaceutical composition containing T lymphocytes as the active substance, prepared by recovering the leukocytes from mammal whole blood, mammal spleen, or mammal thymus gland, or autoblood, separating the leukocytes on nylon wool, and recovering the concentrated T lymphocyte suspension.

2. The pharmaceutical composition according to claim 1, prepared by recovering the leukocytes by centrifugation of mammal blood or autoblood and separating the leukocyte boundary layer.

3. The pharmaceutical composition according to claim 1, prepared by recovering the leukocytes by filtration of mammal whole blood or autoblood, to which aqueous trisodium citrate solution has been optionally added as an anticoagulant, on a cellulose filter, washing the filter with PBS, centrifuging the obtained leukocyte/thrombocyte suspension, and separating the leukocyte boundary layer.

4. The pharmaceutical composition according to claim 1, prepared by recovering the leukocytes from mammal whole blood or autoblood by means of leukopheresis.

5. The pharmaceutical composition according to claim 1, prepared by recovering the leukocytes by leaching mammal spleen or mammal thymus gland with PBS, to which trisodium citrate has been optionally added, centrifuging the obtained solution, and separating the leukocyte boundary layer.

6. A process for the preparation of a pharmaceutical composition according to claim 1 in that the T lymphocytes are formulated in an appropriate administration form.

7. Use of a T lymphocyte suspension for the preparation of drugs for the treatment of viral and bacterial infections, which is prepared by recovering the leukocytes from mammal whole blood, mammal spleen, or mammal thymus gland, or autoblood, separating the leukocytes on nylon wool, and recovering the concentrated T lymphocyte suspension.

8. The use of a T lymphocyte suspension according to claim 7 for the preparation of drugs for the treatment of AIDS and ARC.

## Revendications

1. Composition pharmaceutique contenant, comme matière active, des T-lymphocytes et préparée par une extraction des leucocytes du sang pur d'un mammifère, de la rate d'un mammifère ou du thymus d'un mammifère ou du sang propre; par une coupure desdits leucocytes par l'intermédiaire de la laine nylon, et par une extraction de la suspension de T-lymphocytes enrichie.

2. Composition pharmaceutique selon Revendication 1 et préparée par une extraction desdits leucocytes au moyen d'une centrifugation du sang pur d'un mammifère ou du sang propre et par une séparation de la couche limite de leucocytes.

3. Composition pharmaceutique selon Revendication 1 et préparée par une extraction desdits leucocytes au moyen d'une filtration du sang pur d'un mammifère ou du sang propre, auquel, comme anticoagulant, de la solution citrate trisodique aqueuse est, le cas échéant, ajoutée, par l'intermédiaire d'un filtre cellulosique; par un lavage dudit filtre avec PbS; par une centrifugation de la suspension de leucocytes/thrombocytes obtenue, et par une séparation de la couche de leucocytes.

4. Composition pharmaceutique selon Revendication 1 et préparée par une extraction desdits leucocytes du sang pur d'un mammifère ou du sang propre au moyen d'une leucophérèse.

5. Composition pharmaceutique selon Revendication 1 et préparée par une extraction desdits leucocytes par l'intermédiaire d'un lavage de la rate d'un mammifère ou du thymus d'un mammifère avec PbS, auquel du citrate trisodique est, le cas échéant, ajouté; par une centrifugation de la solution obtenue et par une séparation de la couche de leucocytes.

6. Procédé pour la préparation d'une composition pharmaceutique selon Revendication 1, caractérisé en ce que les T-lymphocytes sont incorporés dans une forme de présentation appropriée.

7. L'utilisation d'une suspension de T-lymphocytes pour la préparation des médicaments pour le traitement des infections virales et bactériennes, ladite suspension étant produite par une extraction des leucocytes du sang pur d'un mammifère, de la rate d'un mammifère ou du thymus d'un mammifère ou du sang propre; par une coupure desdits leucocytes par l'intermédiaire de la laine nylon, et par une extraction de la suspension de T-lymphocytes enrichie.

8. L'utilisation d'une suspension de T-lymphocytes selon Revendication 7 pour la préparation des médicaments pour le traitement du SIDA et des complexes étant relatifs au SIDA.
